# EUROPEAN PATENT APPLICATION

(11) **EP 0 699 904 A1**
(43) Date of publication of application: **06.03.1996**
(21) Application number: 95113759.5
(22) Date of filing: 01.09.1995
(51) Int. Cl.: G01N 33/50

(54) **Method for detecting organopathyinducing factors**

(30) Priority: 02.09.1994 JP 234321/94
(71) Applicant: Kureha Chemical Industry Co., Ltd., Tokyo 103 (JP); TOKYO WOMEN'S MEDICAL COLLEGE, Tokyo 162 (JP)
(72) Inventor: Yoshioka, Toshimasa, Shinjuku-ku, Tokyo 162 (JP); Ito, Katsumi, Shinjuku-ku, Tokyo 162 (JP); Motojima, Masaru, c/o Kureha Chem. Ind. Co., Ltd., Shinjuku-ku, Tokyo 169 (JP)
(74) Representative: Cohausz & Florack

(57) **Abstract**

A method for detecting an organopathy-inducing factor in a serum or plasma sample, comprising the steps:
(1) cultivating a cell from a cultured cell line derived from an organ corresponding to an organ for which disorder is to be determined, in the presence of said serum or plasma sample, whereby reactive oxygen species are released into a culture medium;
(2) adding a spin-trap agent to said culture medium obtained from step (1); and
(3) measuring a concentration of radicals in said culture medium obtained from step (2) is disclosed. An organopathy can be detected at an early stage.

## Description

### BACKGROUND OF THE INVENTION

### 1. Field of the Invention

The present invention relates to a method for detecting organopathy-inducing factors, which can be used for the diagnosis of organopathy. More particularly, it relates to a method for obtaining indicators of organopathy by Electron Paramagnetic Resonance (EPR) (or Electron Spin Resonace, ESR) or the like, which enables one to detect organopathy-inducing factors and to diagnose even latent organopathy at an early stage.

### 2. Description of the Related Art

Hitherto, clinical diagnosis of organopathy was not carried out until the organopathy actually became clear. In organ incompetency, the parameters indicating organ functions are abnormal in value after the organopathy has advanced to a certain extent. When the organopathy is manifested, it is often impossible to stop the advance of the disorder. For example, when renal dysfunction has progressed to a certain level, clinical diagnosis of renal failure can be done by a rise in serum creatinine, which is an index of glomerular filtration rate (i.e. renal function), or by histological changes in the kidney. In particular, in acute renal failure, it is often impossible to stop the advance of disorder when renal dysfunction has become clinical (such oliguria, edema).

Attempts have been made to use the EPR of the blood for the diagnosis of inflammation, external trauma, allergies, collagenosis, hepatocholangitic diseases, diseases of the circulatory system, malignant tumors, pregnancy and other conditions and states [Fukumi Morishige, et al. Igaku-to-Seibutsugaku (Medicine and Biology), 96(6) 451-456 (1978)]. Further, there is a free radical measurement kit and measurements by EPR using the same kit were reported [Japanese Unexamined Patent Publication (Kokai) No. 3-211460]. Nevertheless, attempts to use the EPR signal of the blood for diagnosis of various types of diseases were made for studies at the time when the disease manifests itself externally.

In recent years, the relationship between reactive oxygen species (ROS) including free radicals and disease has attracted attention. In cells wherein oxygen is used to produce energy, intermediary metabolites resulting from oxygen molecules, namely, ROS, such as superoxide anions (O₂⁻·), hydrogen peroxide (H₂O₂), hydroxyl radicals (HO·), hydroperoxyl radicals (HOO·), singlet oxygen (¹O₂), are produced by successive reductions of an oxygen molecule. ROS are known to pay a beneficial role in the sterilizing process in phagocytes. However, it is also known that ROS has a close relationship with various organopathies such as aging, cancer, inflammation, ischemic organopathy, arteriosclerosis, and drug-induced diseases [Kunie Nakamura, et al., Kassei-Sanso-Furii-Rajikaru (J.Act. Oxyg. Free Rad.), 3(1); 63-70, 1992]. Research on the relationship between ROS and disease, however, related to the mechanism of the disease. Application of said relationship has not been known.

### SUMMARY OF THE INVENTION

The present inventors engaged in intensive research to develop a screening method for detecting organopathy in its early stage. Blood contains causative substances (for example, endotoxins, nephrotoxic drugs, immunocomplexes, etc.) inducing, for example, nephropathy, that is, nephropathy-inducing factors. The inventors discovered that it is possible to detect nephropathy-inducing factors contained in the blood, using the fact that ROS is released from kidney cells at the early stage of nephropathy, namely, by applying serum or plasma to cultured kidney cells to thereby cause the serum or plasma to act on the cells, and measuring the release of ROS from the cells by means of EPR or the like. Further, the inventors discovered that specific organopathy-inducing factors can be detected by causing serum or plasma to act on specific cultured organ cells other than cultured kidney cells and measuring the release of ROS from the cells by means of EPR or the like. The present invention is based on these discoveries.

Therefore, the object of the present invention is to provide a method for detecting organopathy in its early stage.

Other object and advantages of the present invention will be apparent from the following description.

In accordance with the present invention, there is provided a novel method for detecting organopathy-inducing factors in a serum or plasma sample, comprising the following steps (1) to (3):
(1) cultivating a cell from a cultured cell line derived from an organ corresponding to an organ for which disorder is to be determined in the presence of the serum or plasma sample to thereby release ROS into a culture medium,
(2) adding a spin-trapping agent to the culture medium obtained from step (1), and
(3) measuring a concentration of radicals in the culture medium obtained from step (2).

### BRIEF DESCRIPTION OF THE DRAWINGS

Fig. 1 shows EPR spectra obtained when Dulbecco's phosphate buffered saline (D-PBS) (control) and E. coli-derived lipopolysaccharide (endotoxin: type 0111, 10 µg/ml) were allowed to act on immortalized bovine glomerular endothelial cells,
Fig. 2 shows EPR spectra obtained one hour after adding sera from a patient (three-year-old male child) suffering from hemolytic uremia syndrome (HUS) in the acute stage and convalescent stage to immortalized bovine glomerular endothelial cells,
Fig. 3 shows EPR spectra of free radicals produced from immortalized glomerular endothelial cells by the serum from a patient (16-year-old male) suffering from acute allograft rejection after a kidney transplant in the early stage of the rejection and by the serum after treatment of the rejection,
Fig. 4 is a graph showing the changes in the serum urea-nitrogen and serum creatinine for a patient suffering from rhabdomyolysis, from the day when entered the hospital to the 5th day in hospitalization, and
Fig. 5 is a graph showing the changes in the amount of free radicals produced from immortalized bovine glomerular endothelial cells by the serum of a patient suffering from rhabdomyolysis.

### DESCRIPTION OF THE PREFERRED EMBODIMENTS

In the present specification, the term "organopathy-inducing factor" means a causative substance which is present in the blood and induces organopathy. The organ is not particularly limited and may be the kidney, liver, heart, lung, digestive tract (stomach and intestines), nervous system (such as brain), or the like. The kind of the disorder is also not particularly limited. Accordingly, for example, a nephropathy-inducing factor is a causative substance which is present in the blood and induces nephropathy, for example, an endotoxin, nephrotoxic drug, immunocomplex, or the like. Further, as other types of organopathy, there may be mentioned hepatopathy-inducing factors, cardiopathy-inducing factors, pulmopathy-inducing factors, gastroenteropathy-inducing factors, encephalopathy-inducing factors, or the like. The method of the present invention may be applied to any causative substance which is present in the blood and induces organopathy. Accordingly, the organopathy-inducing factors in the present invention naturally include substances which are not yet known to exist at the present time.

The steps in the method of the present invention will be explained hereinafter:

### Step (1) : Cultivation of Cultured Organ Cell Lines In Presence of Serum or Plasma to be Examined

Step (1) comprises adding a sample, that is, a serum or plasma, to the cultured cell lines and further cultivating the cell lines whereby reactive oxygen species (ROS) are released in the culture medium.

As the sample, either of serum or plasma may be used. The serum or plasma added as the sample is not particularly limited, so long as it possibly might contain an organopathy-inducing factor to be measured. The sample may be obtained, for example, by separating the serum or plasma from blood taken from humans or animals by ordinary methods. The amount of serum or plasma added may be 0.1 to 1.0 ml, preferably 0.4 to 0.6 ml, per one examination with respect to the cell numbers (1 x 10⁶ to 10 x 10⁶) used in a usual examination. If the amount of serum or plasma added is less than 0.1 ml, the reaction becomes weak and the sensitivity of detection falls. If the amount of serum or plasma added exceeds 1.0 ml, it becomes necessary to increase the amount of the trapping agent used, but the sensitivity does not rise. Thus, it is of no practical use.

As the cultured organ cell lines, cultured cell lines derived from the organ same as the organ in which the disorder is caused by the organopathy-inducing factor to be examined, that is, cultured cell lines derived from the organ corresponding to the organ for which the disorder is to be determined, are used. As the cultured organ cell lines, a cell strain obtained by taking cells from the organ tissue in an animal and subculturing them in accordance with the ordinary tissue culture method may be used. It is preferable to use a so-called immortalized cell strain which permanently sustains considerable growth activity to the extent that the first generation cells possess and further does not lose the form and function inherent to the cells even after the subculturing. Such cells for some organs are also commercially available.

However, when such cells are not commercially available, there are methods for obtaining the immortalized cell strain as follows: (1) the method comprising introducing into animal cells oncogene, such as ras or c-myc, or DNA type tumor virus gene, such as adenovirus E1A, SV40 virus, human papilloma virus (HPV16), or their tumor antigen (T-antigen) gene, and subculturing the transformant; (2) the method comprising preparing a transgenic animal having a oncotic gene or its T-antigen gene stably incorporated in a part of the chromosome, obtaining the primary cells from the transgenic animal which holds a part of the oncotic gene in the somatic cells and/or germ cells already at the time of ontogeny, and subculturing the same; (3) the method comprising introducing the temperature-sensitive mutant SV40 large T-antigen gene into a totipotent cell of a mammal, obtaining the transgenic animals by normal reproduction of said mammal, taking the cells from the tissue of the various organs in said mammal and subculturing the same; and so on.

As the cultured kidney cells it is preferable to use cell lines from endothelial cells, immortalized bovine glomerular endothelial cells, glomerular mesangium cells, LLCPK1 cells (a cell line derived from renal tubules, i.e., the common loci of acute renal failure), or the like. It is particularly preferable to use the immortalized bovine glomerular endothelial cell lines, because the cultivation thereof is simple and the cell lines can be subcultured substantially permanently.

The cell cultivation in the step (1) of the present invention may be performed by ordinary methods in an ordinary cell culture facility, for example, a clean bench (SCV1303EC2A; Hitachi, Tokyo), or the like. The subculturing cells may be maintained in a culture flask (for example, 75 cm; plastic flask made by Corning or Falcon). The culture medium and culture conditions may be suitably selected in accordance with the organ tissue used. For example, when cultivating renal glomerular cells, it is preferable to use RPMI1640, or the like as the culture medium.

The cultured cell lines used may be subcultured on a culture plate (for example, having six wells of a diameter of 35 mm, e.g. 6-well plate; made by Corning or Falcon) coated with, preferably, gelatin (for example, made by Sigma) The cells for subculture in the culture flask are separated with an enzyme (for example, trypsin), suspended in the culture medium, then divided on the culture plate. After, 24 to 72 hours, the cell density becomes constant (approximately 100,000 to 500,000 cells/well on the 6-well plate), the cells are further cultivated for 12 to 72 hours in a maintaining medium (for example, RPMI 1640 containing 0.5% fetal bovine serum).

Cells from the cultured cell lines can be stimulated with a serum or plasma sample by the following method: For example, the cells on the culture plate at the step (1) of the present invention first are washed with a medium such as Dulbecco's PBS (D-PBS including calcium and magnesium; Gibco) or the like. Then, a given amount of D-PBS and sample are added to each plate. Typically, 0.4 ml of D-PBS and 0.2 ml of sample are applied to a well of 6-well plate. Subsequently, the cells are cultivated in a CO₂ incubator (a normal incubator for cell culture, for example, WJ-3C, Hirasawa Seisakusho, Tokyo) or the like. The preferable cultivation or incubating period is 0.5 to 5 hours, more preferably 1 to 2 hours, most preferably 1 hour. If the incubation period is shorter than 0.5 hour, no reaction occurs, while if it is longer than 5 hours, the cells sometimes die.

If there exists in the serum or plasma sample, an organopathy-inducing factor which may cause a disorder of the organ from which the cultured organ cell lines are derived, ROS is released in the culture medium during the cultivation. The term "ROS" includes a superoxide anion, hydrogen peroxide, hydroxyl radical, singlet oxygen, and so on produced by the incomplete reduction of the oxygen molecules.

### Step (2) : Addition of Spin-Trapping Agent

Step (2) comprises adding a spin-trapping agent to the culture medium obtained by the above step (1). In the step (2), the unstable free radicals, that is, ROS, are reacted with the spin-trapping agent to convert them to stable, easily measurable spin adducts.

The spin-trapping agent is not particularly limited so long as it can convert ROS to spin adducts. For example, it is preferable to use 5,5-dimethyl-1-pyrroline-N-oxide (DMPO), 2-methyl-2-nitrosopropane dimer (MNP), (α-phenyl-N-t-butylnitron (PBSN), or the like. Of these spin-trapping agents, DMPO is most preferable, because it is low cytotoxic. The spin-trapping agent is preferably added in excess so that all the ROS in the culture medium can be converted to spin adducts. The reaction may be carried out 1 to 60 minutes, preferably 10 to 30 minutes, after adding the spin-trapping agent. If the reaction time is less than 1 minute, conversion to spin adducts is insufficient, while if the reaction time is over 60 minutes, the spin-trapping agent may cause cell dysfunction.

### Step (3) : Measurement of Concentration of Radicals

Step (3) comprises measuring the concentration of radicals in the culture medium obtained at the step (2), for example, using an EPR apparatus.

It is possible to perform the measurement by setting suitable conditions in accordance with the radicals to be measured using an ordinary EPR apparatus. The frequency may be any of 1.2 GHz (L-band), 9.4 GHz (X-band), 24 GHz (K-band), 36 GHz (Q-band), or the like, but the X-band is preferable. As the spin measurement standard, for example, 4-hydroxyl-2,2,6,6-tetramethyl-1-piperidine-N-oxyl (4-hydroxy-TEMPO) may be used. The height obtained by integrating the resulting spectrum twice is the concentration of radicals. The radical species can be determined by the shape of the spectrum.

In the method of the present invention, it is possible to automatically determine the possibility of organopathy from the value obtained by measuring the concentration of radicals. According to the method of the present invention, it is possible to predict organopathy. More particularly, it is considered that, when organopathy is caused by factors in the blood, such factors appear prior to the actual disorder. Normal serum or plasma does not contain such factors, and thus no free radicals will be produced even if such a serum or plasma is brought into contact with the cells of the cultured organ cell lines. Only when the serum or plasma contains organopathy-inducing factors, free radicals are produced from the cultured cells. Accordingly, it is possible to automatically detect the possibility of a subject suffering from organopathy, that is, the existence of organopathy-inducing factors in the blood. Using the method of the present invention, it is possible to screen a high risk group with a high probability of disorders from a group of patients having the risk of organopathy.

The method for detecting of organopathy-inducing factors according to the present invention can be carried out with a small amount of a serum or plasma sample. Therefore, the present method can be used for infants or for experiments of small animals in which available sample quantity is limited. Further, because the results can be obtained in a short time, the present method is useful even for acute diseases such as acute renal failure where the state of the disease rapidly changes. The measuring procedure is simple and can be carried out in a short time, and thus it is possible to treat a large amount of samples.

It is considered that the method of the present invention is clinically most useful where organ failure can be diagnosed in patients whose organopathy has been anticipated, before it becomes clear. Transplantation of an organ, such as heart, liver, or kidney, is internationally established as the treatment of organ failure, and will be accepted in Japan. The most important complication in organ transplantation is an allograft rejection. Acute rejection in particular is a complication often caused shortly after transplantation. The onset is rapid and, further, there is less chance of a complete recovery if treatment is started when organ failure is advanced. Therefore, early diagnosis is necessary. The acute rejection which occurs after a kidney transplantation, hitherto, was diagnosed after the value of the serum creatinine (an index of kidney function) was increased, and then treatment started. The rise of the serum creatinine value occurs only after the decline in the kidney function proceeds to a certain extent. Also, the value fluctuates even when the kidney is normal and it is sometimes difficult to distinguish if a slight rise in the creatinine value is a normal fluctuation or reflections of the early stage of rejection. Therefore, diagnosis by some other method has been desired. The present invention provides a means of solution of this problem.

For example, in the acute rejection, the lymphocytes in blood are activated and the bioactive substances known as cytokines are released. The bioactive substances promote the release of free radicals from the endothiel cells. Therefore, for example, using the free radical release reaction of cultured glomerular endothelial cells, caused by a serum or plasma sample, according to the present invention, it is possible to diagnose an early acute rejection reaction.

The method for detecting organopathy-inducing factors according to the present invention have the above characteristics, and is useful as a novel method for screening organopathy, preferably nephropathy, using EPR, and, further, is useful as a novel method for screening potential organopathy, preferably, potential nephropathy at the early stage. For example, the screening method of the present invention may be applied to a group examination to find nephropathy at the early stage.

Further, if the method for detecting organopathy-inducing factors according to the present invention is carried out for cells from various kind of organs as the cultured cells, it is possible to determine the organ where the organopathy occurs.

### Examples

The present invention will now be further illustrated by, but is by no means limited to, the following Examples.

### Example 1 : EPR Patterns in Immortalized Bovine Glomerular Endothelial Cells Treated with Nephropathy-Inducing Factor

As the nephropathy-inducing factor, the E. coli toxin, lipopolysaccharide (LPS) (E. coli. 0111; Sigma) was used.

Immortalized bovine glomerular endothelial cells were prepared by the method described in Nitta K., et al., Jpn. J. Nephrol., 1994; 36: 883-889.

Bovine glomerular endothelial cells (GEN) were separated from mature bovine kidneys, cloned, and proliferated by the modified method disclosed in Nitta K., et al., Acta Pathol., Japonica, 1993; 43: 367-371 in accordance with the method disclosed in Nitta K., et al., J. Am. Soc. Nephrol., 1991; 2: 156-163. The separated GEN was maintained in a medium A. The medium A was prepared by adding to RPMI 1640 medium (Gibco Oriental Co., Tokyo, Japan) 15% heat-inactivated fetal calf serum (FCS), 5 U/ml heparin, 2 ng/ml acidic fibroblast growth factor (FGF; R&D System; Minneapolis, USA), and antibiotics.

Plasmid DNA for expressing the SV40 large T-antigen (Brash DE et al., Mol. Cell Biol., 1987; 7: 2031-2034) was introduced into E. coli and proliferated. The plasmid DNA was separated by a Qiagen column. The eluted DNA was precipitated with ethanol and resuspended in sterile TE buffer (10 mM Tris-HCl, 1 mM EDTA, pH 8.0). Then, the absorbance at 260 nm was measured to determine the amount of DNA.

The DNA was introduced into the bovine GEN by the liposome mediated method which is a modified method of the protocol of Ponder KP, et al., Human Gene Therapy, 1991; 2: 41-52 and Gibco BRL (Gaithersburg, USA).

More particularly, the GEN was placed on a gelatin-coated six-well plastic culture plate in an amount of 2 x 10⁴ cells/well. A cellular monolayer was formed in the medium A after 12 hours.

The liposome/DNA mixture was prepared by mixing 15 µg of lipofectin (Gibco BRL) and a predetermined amount of the DNA prepared above in 50 µl of distilled water. The resulting mixture was incubated at room temperature for 15 minutes, and then, added to the cultured GEN. The all preparations were incubated in 5% CO₂ at 37°C for 18 to 20 hours. Then, the culture medium was replaced with a fresh medium A and the GEN was cultured for further 48 hours.

Further, the culture medium was replaced with a selective medium. The select medium was prepared by adding to RPMI 1640 medium 10% FCS, ITS Premix (5 µg/ml insulin, 5 µg/ml transferrin, and 5 ng/ml selenium), and antibiotics. Cells which actively proliferated were selected using the usual cloning method. One clone was selected as the clone for proliferation and transferred to a 75 cm culture flask (Corning Glass Works; Corning, USA). The clone was cultured in an RPMI 1640 medium containing 10% FCS and antibiotics (medium B) and subcultured with 0.125% trypsin.

The resulting immortalized bovine glomerular endothelial cells were subcultured on a 6-well culture plate (well diameter = 35 mm; Corning or Falcon) coated with gelatin (Sigma). More particularly, the immortalized GEN in the 75 cm culture flask was separated with trypsin, suspended in the culture medium, and divided on the culture plate. After 24 to 72 hours, the cell density became constant (about 200,000 cells/well), and the cells were further cultured for 48 hours in RPMI 1640 containing 0.5% fetal bovine serum. The culture medium used was RPMI 1640 (for example, the medium made by Gibco, ICN, or Sigma) which contained fetal bovine serum (15%), penicillin (100 µg/ml), streptomycin (100 µg/ml), and Fungizone (0.25 µg/ml) (see Yoshioka et al., Kidney International 35: 211-219, 1994).

The immortalized bovine glomerular endothelial cells cultured in the 6-well plate were first washed with D-PBS (D-PBS containing calcium and magnesium; Gibco). Then, a solution of lipopolysaccharide (LPS; 10 µg/ml), which usually appears in blood at the time of infection by E. coli and causes an acute renal failure, dissolved in D-PBS (0.6 ml) was added and the solution was incubated for 1 hour in a CO₂ incubator (cell culture incubator: Hirasawa Seisakusho WJ-3C; Tokyo). Then, the spin-trapping agent, DMPO (Labotec, Tokyo; 0.45M, 30 µl) was added and a reaction was performed for 20 minutes. As a control test, D-PBS not containing lipopolysaccharide was added to the cultured cells. After DMPO was similarly added, the reaction was performed for 20 minutes.

After the reaction was completed, the culture medium was taken in 13 x 75 mm glass test tubes (Maruem, Osaka). Then, the EPR pattern (differentiated form) of the sample was immediately determined by an EPR apparatus (JES-RE1X, Nippon Denshi). The measurement conditions were a gain of 5 x 100, a time constant of 0.1 second, a sweep of 5 mT, and a magnetic field modulation of 0.63 x 10⁻¹ mT. Further, as the spin measurement standard for determining the concentration of free radicals, a standard solution (10⁻⁶M) of 4-hydroxy-TEMPO (made by Sigma) was prepared. The EPR pattern was measured under the conditions same as those for the sample. From a comparison of the signal intensity (integrated value of the spectrum) obtained, the spin count was measured using a Hewlett-Packard personal computer (9145, Stoneham, USA) and EPR analysis software (Nippon Denshi ESROUT 470 data system). The resulting spectrum was printed out by a Hewlett-Packard plotter (7475A).

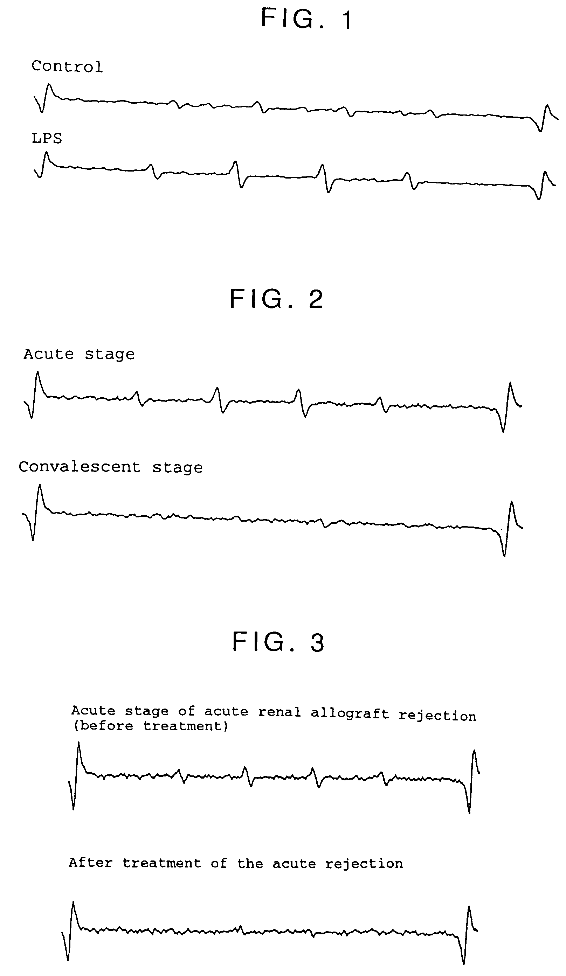

The two types of EPR patterns obtained from the above experiments are shown in Fig. 1. The top pattern (control) of Fig. 1 shows the results of the control experiment, while the bottom pattern (LPS) shows the results of the experiment in which E. coli-derived lipopolysaccharide (LPS) was added. The two spectra at both ends in each pattern are standard waves incorporated in the machine (Mn+ spectrum). The height of the standard waves and the height of the waves obtained from the specimen indicate the relative radical concentration. The concentration was calculated from the standard line of 4-hydroxy-TEMPO. In Fig. 1, the four waveforms observed in the pattern in the case of addition of E. coli-derived lipopolysaccharide (LPS) were spectra characteristic of hydroxyl radicals. Accordingly, as clear from the two patterns of Fig. 1, hydroxyl radicals were detected in the culture medium of the immortalized bovine glomerular endothelial cells to which one of the causative substances of acute renal failure, LPS, was added. Only minor waves were observed in the control pattern. The concentrations of free radicals in the control test and the LPS-added test were 0.21 and 4.6 nmol/10⁷ cells, respectively.

### Example 2 : EPR Patterns in Immortalized Bovine Glomerular endothelial Cells Treated with Serum of Patient Experiencing From Acute Renal Failure

The sera of a patient (three-year-old male child) with acute renal failure of hemolytic uremia syndrome (HUS) due to E. coli 0157 in the acute stage (an early stage of renal failure) and convalescent stage (the condition when the renal failure was recovered) were used to examine the production of free radicals from cultured cells.

In the present experiment, 0.4 ml of the serum sample and 0.2 ml of D-PBS were mixed and the mixture was brought into contact with the immortalized bovine glomerular endothelial cells. Then, the EPR patterns were obtained in the same way as in Example 1. As shown in Fig. 2, the release of hydroxyl radicals was observed in the immortalized bovine glomerular endothelial cells treated with the acute stage serum [top part of Fig. 2 (acute stage)], while no free radicals were detected by the serum of the convalescent stage [bottom part of Fig. 2 (convalescent stage)]. The amount of free radicals produced by the acute stage serum was found to be 2.4 nmol/10⁷ cells. Further, no free radicals were detected when the acute stage serum was not contacted with the cells. This means that there exist, in the serum, factors (nephropathy inducing factors) which act on cells and promote the release of free radicals from the endothelial cells.

From the results of the above experiments, it is clear that the method of the present invention is useful for detection of nephropathy-inducing factors.

### Example 3 : EPR Patterns in Immortalized Bovine Glomerular Endothelial Cells and LLCPK1 Cells Treated with Serum of Patient with Acute Renal Allograft Rejection After Kidney Transplantation

In the present Example, the reactions of the initial lesions (the endothelial cells) of the rejection reaction, and the secondary lesions, (uriniferous tubular epithelial cells) of the rejection reaction, with the serum were compared using immortalized bovine glomerular endothelial cells and immortalized renal tubular epithelial cells, that is, LLCPK1 cells. The LLCPK1 cells (Dainippon Seiyaku), as described in the specifications were subcultured in an RPMI 1640 medium containing a 10% FBS. The cells used for the experiment were subcultured on a six-well culture plate in the same way as the immortalized bovine glomerular endothelial cells.

In the present Example, the serum of the early stage of the acute rejection reaction after a kidney transplantation, that is, the acute stage (active) serum, and the serum after treatment of the rejection reaction, that is, the convalescent stage (inactive) serum, of a patient (16-year-old male) who developed acute allograft rejection reaction were applied to immortalized bovine glomerular endothelial cells in the method same as in Example 2, and the production of free radicals was measured by the EPR. As shown in Fig. 3, comparing the top part of Fig. 3 [acute stage of acute allograft rejection (before treatment)] and the bottom part [after treatment of the rejection], it is clear that factors causing the production of hydroxyl radicals from the endothelial cells are contained in the serum already at the early stage of the rejection. That is, it became clear that it is possible to detect the organ dysfunction due to acute rejection, using immortalized bovine glomerular endothelial cells, because production of hydroxyl radicals is clearly observed in the cells treated with the acute stage serum (determined as 1.28 nmol/10⁷ cells), while no production of free radicals at all is observed in the convalescent stage serum. Similar results were obtained when plasma samples were used. On the other hand, in the LLCPK1 cells, only slight production of free radicals due to the acute stage serum was observed (0.018 nmol/10⁷ cells), (whereas free radicals were not detected when the convalescent stage serum was used). The lesions in the initial stage of the acute rejection were considered to be at the endothelial cells. In the EPR pattern, free radicals from endothelial cells were strongly detected - about 100-fold stronger compared with tubular epithelial cells. Accordingly, it is manifest that it is possible to predict the portions with a high risk of disorder in the organ and possible to predict the organs or portions where the multiple organopathy-inducing factors in the blood cause a disorder with a high risk, using the cultured cells of various organs.

### Example 4 : Detection of Free Radicals Produced From Immortalized Bovine Glomerular Endothelial Cells by Serum of Acute Renal Failure Caused by Rhabdomyolysis

One of the causes of acute renal failure is rhabdomyolysis which prevailed after the Great Hanshin Earthquake in January 1995. Rhabdomyolysis is a disease which causes lysis of myocytes due to the physical exertion (such as physical training or crush syndrome) in a person who has been healthy. In this Example, the indicator of kidney dysfunction and release of free radicals from cultured cells stimulated by the serum from the patient (12-year-old boy) were observed over time in rhabdomyolysis caused by baseball training.

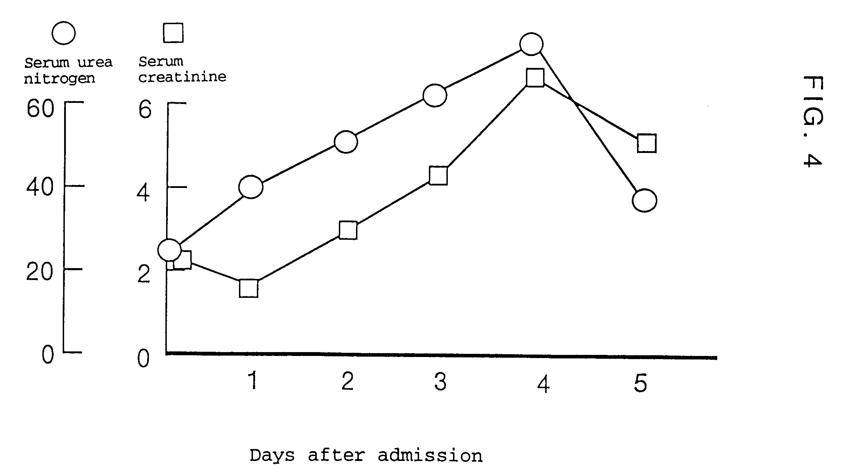

As clear from Fig. 4 showing the results, on days after 1 and 2 days from the disease, no remarkable rise in the urea nitrogen (○) and creatinine (□) foreshadowing strong renal dysfunction necessitating dialysis was observed. Further, it is believed that the urea nitrogen and creatinine values rise along with the fusion of muscles in rhabdomyolysis. The urea nitrogen and the creatinine are contained in the protein of the muscle. Therefore, even if the kidney function does not fall, the concentration of the serum rises along with destruction of muscle. In the initial stage, these values cannot indicate the accurate kidney function. In particular, the fluctuations in the creatinine value did not suggest nephropathy. Further, while not shown in Fig. 4, the amount of urine was maintained. Thus, diagnosis of the early stage of nephropathy was difficult. Subsequently, the urea nitrogen and creatinine value rose and the acute renal failure became apparent. The patient was dialyzed on the days 4 and 5 due to severe impairment in renal function.

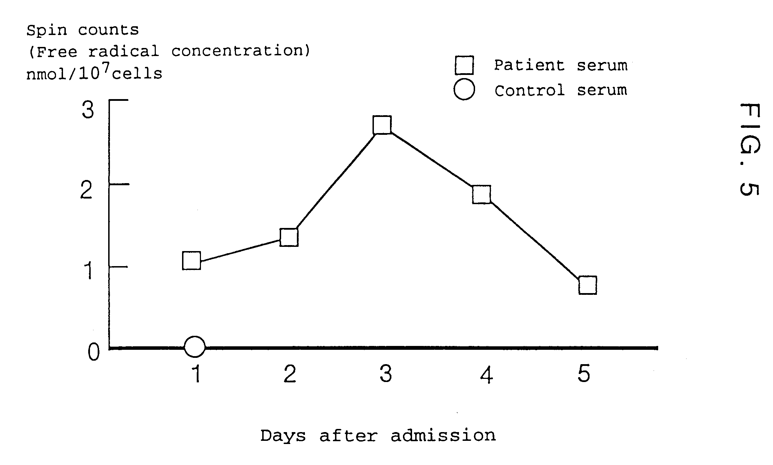

Further, in the present Example, in the same way as in Example 2, 0.1 ml of patient's serum was applied to immortalized bovine glomerular endothelial cells together with 0.5 ml of D-PBS, 30 µl of DMPO were added after one hour, and then the concentration of free radicals was measured. The results are shown in Fig. 5. As clear from Fig. 5, no radicals at all were detected in the control serum (○), whereas in the patient's serum (□), production of over 1 nmol/10⁷ cells of free radicals was already observed on the days after 1 and 2 days of the disease when the nephropathy was still not clear. In the present Example, the blood was dialyzed on the days after 4 and 5 days of the disease. A fall in the urea nitrogen and the creatinine values as well as a reduction in the production of free radicals due to introduction of hemodialysis were observed.

Accordingly, the present Example shows that the method of the present invention is a useful method of diagnosis even when the organopathy is not apparent or when the nephropathy cannot be evaluated by ordinary indicators.

As above the method of the present invention is completely novel, because it can measure the organopathy-inducing factors, which were never measured by conventional methods. Thus, conventional methods can measure each factor separately, however, organ failrure often develop from multiple factors. The present method can determine overall potency for sum of the factors. According to the method of the present invention, it becomes possible to judge the risk that a patient may develop organopathy, at an early stage, when the blood is taken.

Further, the method of the present invention is useful for screening organopathy, preferably nephropathy using EPR, and is useful for screening potential organopathy, preferably potential nephropathy in the early stage.

Although the present invention has been described with reference to specific embodiments, various changes and modifications obvious to those skilled in the art are deemed to be within the spirit, scope, and concept of the invention.

## Claims

1. A method for detecting an organopathy-inducing factor in a serum or plasma sample, comprising the steps:
(1) cultivating cells from a cultured cell line derived from an organ corresponding to an organ for which disorder is to be determined, in the presence of said serum or plasma sample, whereby a reactive oxygen species is released into a culture medium;
(2) adding a spin-trap agent to said culture medium obtained from step (1); and
(3) measuring a concentration of radicals in said culture medium obtained from step (2).

2. The method according to claim 1, wherein said cultured cell line is a cultured kidney cell line.

3. The method according to claim 2, wherein said cultured kidney cell line is derived from a glomerular endothelial cell, a glomerular mesangium cell, or a LLCPK1 cell.

4. The method according to claim 3, wherein said cultured kidney cell line derived from the glomerular endothelial cell is immortalized glomerular endothelial cell line.

5. The method according to claim 1, wherein said organopathy-inducing factor is a nephropathy-inducing factor.

6. The method according to claim 1, wherein said organopathy is a latent organopathy.

7. The method according to claim 6, wherein said latent organopathy is a latent nephropathy.

8. The method according to claim 1, wherein said spin-trap agent is 5,5-dimethyl-1-pyrroline-N-oxide.

9. The method according to claim 1, wherein said cultivating step (1) is carried out for 0.5 to 5 hours.

10. The method according to claim 1, wherein the cultivation is further continued for 1 to 30 minutes after adding said spin-trap agent in the step (2).

11. A method for detecting an organopathy, comprising detecting an organopathy-inducing factor by the method according to claim 1, for one or more cultured cell lines.

12. A method for screening an organopathy at an early stage, comprising detecting an organopathy-inducing factor by the method according to claim 1.
